# EUROPEAN PATENT APPLICATION

(11) **EP 3 047 869 A1**
(43) Date of publication of application: **27.07.2016**
(21) Application number: 15152237.2
(22) Date of filing: 23.01.2015
(51) Int. Cl.: A61M 5/34

(54) **Needle assembly with needle coupling for snap fit on hub**

(71) Applicant: Sanofi-Aventis Deutschland GmbH, 65929 Frankfurt am Main (DE)
(72) Inventor: HOLTWICK, Marc, 65926 Frankfurt am Main (DE); MARX, Wolfgang, 65926 Frankfurt am Main (DE); ZANGERLE, Wolfgang, 5507 Mellingen (CH); HUBER, Roland, 5604 Hendschiken (CH)
(74) Representative: Finger, Catrin

(57) **Abstract**

The invention relates to a needle assembly (4), comprising
a needle hub (3) and
a needle (2) fixed to the needle hub (3),
wherein the needle (2) is fixed to the needle hub (3) by an intermediate component (1), wherein the needle (2) is firmly bonded to the intermediate component (1), and wherein the intermediate component (1) is coupled to the needle hub (3) by a latching connection.

## Description

### Technical Field

The invention relates to a needle assembly. The invention further relates to a method for mounting a needle assembly.

### Background of the Invention

Needle assemblies for injection devices are well known. The process of bonding a needle to a needle hub involves precisely positioning to ensure a mechanically sealed connection between the needle and the needle hub. For example, the needle is bonded to the needle hub by using an adhesive. Therefore, the needle hub may comprise undercuts in the bonding area as adhesive pads to provide a stable and durable connection between the needle and the needle hub. Otherwise, there is a trend toward the use of a needle hub made from a hard plastic material, such as cyclo-olefin-copolymer (COC). COC is bio-compatible, so that it does not alter pre-filled medications as it may be the case with softer plastics such as polypropylene or polyolefin. In the case of a COC needle nub, the needle cannot be bonded to the needle hub by using an adhesive, because COC is too hard to insert undercuts as adhesive pads. Nevertheless, to provide a mechanically sealed connection between the needle and the needle hub, the needle may be inserted into the COC needle hub by ultrasonic welding. During the ultrasonic welding of the needle, particles may release from the COC and get into the flow channel of the needle causing clogging or delivery of a medicament contaminated with COC particles into the patient.

Thus, there remains a need for an improved needle assembly and an improved method for mounting a needle assembly.

### Summary of the Invention

It is an object of the present invention to provide an improved needle assembly and an improved method for mounting a needle assembly.

The object is achieved by a needle assembly according to claim 1 and by a method according to claim 12.

Exemplary embodiments of the invention are given in the dependent claims.

According to the invention, a needle assembly comprises a needle hub and a needle fixed to the needle hub. According to the invention, the needle is fixed to the needle hub by an intermediate component, wherein the needle is firmly bonded to the intermediate component, and wherein the intermediate component is coupled to the needle hub by a latching connection. The intermediate component enables a fixation of the needle to the needle hub without the risk of clogging the needle or contaminating a medicament flowing through the needle by released particles from the needle hub that may be generated by a direct fixation of the needle to the needle hub, i. e. by ultrasound welding.

In an exemplary embodiment, the intermediate component is made from a soft resilient material such as polyolefin and the needle hub is made from a hard plastic material such as cyclo-olefin-copolymer. This soft-hard connection within the needle assembly enables to combine the advantages of a needle hub made from a hard plastic material and a tight fixation of the needle without generating particles that may chip from the hard plastic material and clog the needle.

In a further exemplary embodiment, the intermediate component comprises a snap element adapted to couple the intermediate component to the needle hub. Thus, the intermediate component couples to the needle hub by a snap-on connection without using an adhesive that may be not quite cured at particular points.

In a further exemplary embodiment, the intermediate component comprises a section which forms a press fit between the needle and the intermediate component. The press fit may avoid a direct contact between the medicament in the needle and an adhesive by which the needle is bonded to the intermediate component. Furthermore, the intermediate component may comprise a number of ribs that are arranged on an outer circumference of the intermediate component to provide a press fitting between the intermediate component and the needle hub. In an exemplary embodiment, the ribs are arranged ring-shaped around the outer circumference of the intermediate component.

In an exemplary embodiment, the needle is firmly bonded to the intermediate component by an adhesive such as a light-curable adhesive. In a further embodiment, the intermediate component comprises or is made from a material that transmits ultraviolet rays (=UV). This may provide an effective curing, because less radiation energy is required for curing the adhesive that bonds the needle to the intermediate component. Thus, a time for curing the adhesive is reduced compared to a material that is not able to transmit UV rays. In particular, a curing time may be in a range of a few seconds. Moreover, the UV-permeable material may also reduce a potentially negative UV-effect on the material of the intermediate component.

The invention further provides a method for mounting a needle assembly, comprising the following steps:
- prefixing a needle within an intermediate component,
- firmly bonding the prefixed needle to the intermediate component by applying an adhesive, wherein the adhesive is subsequently cured, and
- snap fitting the intermediate component including the needle to a needle hub.

Because the adhesive is applied and cured before the needle is fixed to the needle hub, the risk for contaminating an area of the needle assembly that may get in contact with a medicament of a medicament delivery device, to which the needle assembly may be coupled, is reduced.

In an exemplary embodiment, the adhesive is cured by ultraviolet rays.

Further scope of applicability of the present invention will become apparent from the detailed description given hereinafter. However, it should be understood that the detailed description and specific examples, while indicating exemplary embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given herein below and the accompanying drawings which are given by way of illustration only, and thus, are not limitative of the present invention, and wherein:
- Figure 1: is a schematic perspective view of an intermediate component in an exemplary embodiment,
- Figure 2: is a schematic perspective view of an intermediate component and a needle during a mounting process in an exemplary embodiment,
- Figure 3: is a schematic perspective exploded view of an exemplary embodiment of a needle assembly comprising a needle hub, the intermediate component and the needle,
- Figure 4: is a further schematic perspective exploded view of an enlarged section with the needle assembly according to figure 3, wherein the intermediate component and the needle are shown in an assembled state,
- Figure 5: is a schematic perspective view of the needle assembly according to figure 4 in an assembled state, and
- Figure 6: is a schematic longitudinal section of an exemplary embodiment of a needle assembly coupled to a medicament delivery device.

Corresponding parts are marked with the same reference symbols in all figures.

### Detailed Description

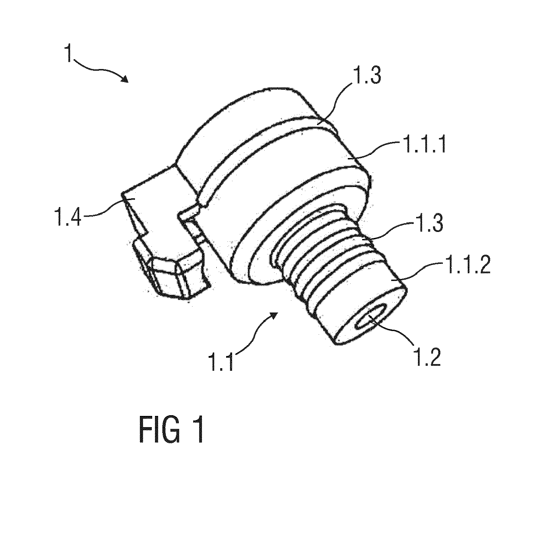

Figure 1 shows an exemplary embodiment of an intermediate component 1 in a schematic perspective view.

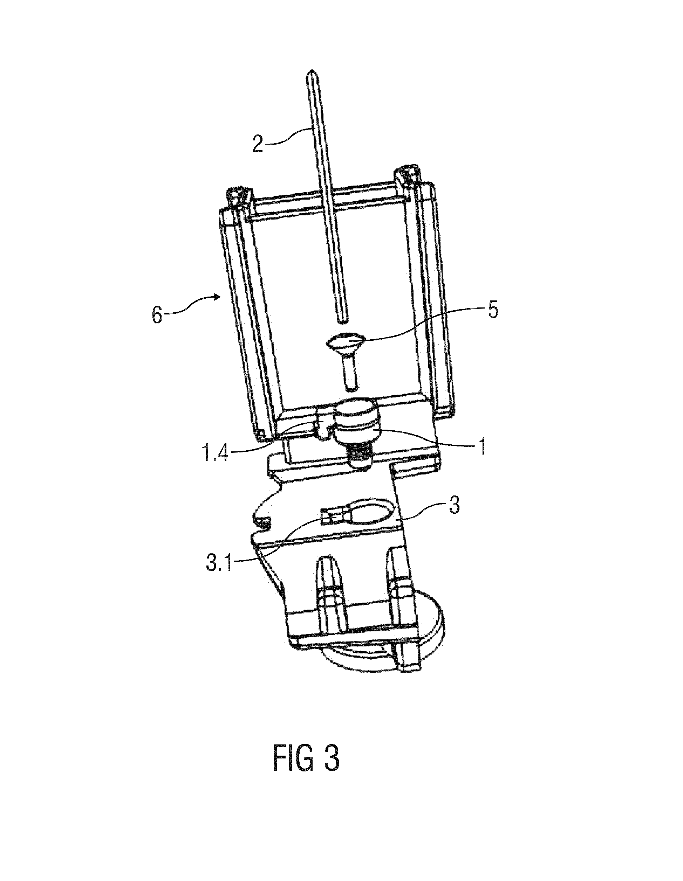

The intermediate component 1 is provided to receive a needle 2 and to couple to a needle hub 3 of a needle assembly 4 that is exemplarily shown in the figures 3 to 6.

The intermediate component 1 is made from or comprises a soft resilient material such as polyolefin or another suitable soft plastic material that particularly transmits ultraviolet rays. According to the shown exemplary embodiment, the intermediate component 1 comprises a substantially cylindrical body 1.1 with two portions 1.1.1, 1.1.2 of different outer diameters. Here, the outer diameter of a first portion 1.1.1 is larger than the outer diameter of a second portion 1.1.2 generating an undercut for an interlocking arrangement of the intermediate component 1 within the needle hub 3 as it is described later.

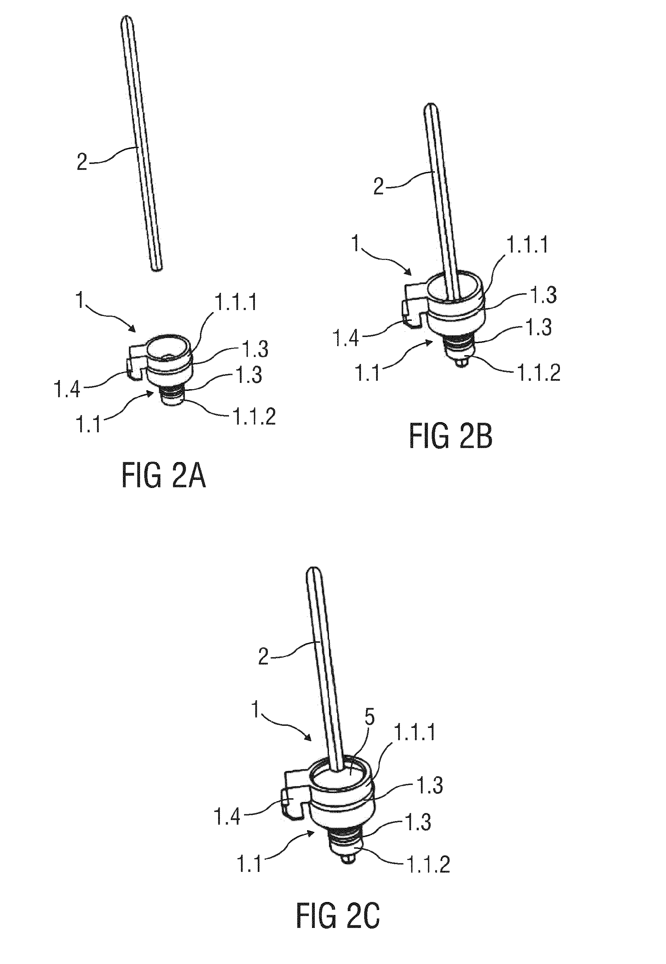

The portions 1.1.1, 1.1.2 are respectively designed as a hollow cylinder generating a continuous opening 1.2 of the intermediate component 1 for receiving the needle 2. Thereby, the opening 1.2 includes a larger diameter in the area of the first portion 1.1.1 and a smaller diameter in the area of the second portion 1.1.2. The different diameters of the opening 1.2 can be seen in comparison in figures 1 and 2.

The first portion 1.1.1 comprises a rib 1.3 that is arranged around the outer circumference of the first portion 1.1.1 at least partially. In the shown exemplary embodiment, the rib 1.3 surrounds the outer circumference of the first portion 1.1.1 excluding an area of a snap element 1.4 that is coupled to the first portion 1.1.1 on a front end side faced away from the second portion 1.1.2. The rib 1.3 and the body 1.1 may be made as one single piece or firmly-bonded together.

In the shown exemplary embodiment, the snap element 1.4 is designed as a latching hook extending partially along a lateral area of the hollow cylindrical first portion 1.1.1, wherein a hooked free end of the snap element 1.4 projects rectangularly away from the lateral area. The snap element 1.4 and the body 1.1 may be designed as one single piece or firmly-bonded together. As regards the latter, the snap element 1.4 may be made from or comprise a plastic material that is harder than the soft resilient material of the body 1.1. Alternatively, the snap element 1.4 and the body 1.1 are made from or comprise a soft plastic material with the same level of hardness. The snap element 1.4 is provided to generate a latching connection between the intermediate component 1 and the needle hub 3.

Furthermore, the second portion 1.1.2 comprises a number of further ribs 1.3 that are arranged around the outer circumference of the second portion 1.1.2. In the shown exemplary embodiment, the second portion 1.1.2 comprises three ribs 1.3 that are arranged spaced to each other in a longitudinal extension of the second portion 1.1.2. Alternatively, the second portion 1.1.2 may comprise less or more than three ribs 1.3. The ribs 1.3 and the body 1.1 may be made as one single piece or firmly-bonded together.

In figure 2, an exemplary mounting process of mounting a needle 2 to the intermediate component 1 is shown. The mounting process comprises three steps a) to c).

In a first step a), the intermediate component 1 and the needle 2 are provided. The needle 2 may be designed as a hypodermic needle comprising a hollow body or a massive body of stainless steel.

In a second step b), the needle 2 is inserted into the opening 1.2 of the intermediate component 1 and thus prefixed to the intermediate component 1.

In a third step c), an adhesive 5 is applied into a space between the needle 2 and the intermediate component 1, in particular between the needle 2 and an inner wall of the first and second portion 1.1.1, 1.1.2. The inner wall of the first portion 1.1.1 and/or the second portion 1.1.2 may comprise a section (not shown) that forms a press fit between the needle 2 and the inner wall in order to avoid a contamination of an medicament outlet of the needle 2 with the adhesive 5.

Subsequently, the adhesive 5 is cured by light such as ultraviolet rays. The application of the adhesive 5 for fixing the needle 2 to the intermediate component 1 outside the needle hub 3 enables a reliable curing of the adhesive 5 at every point.

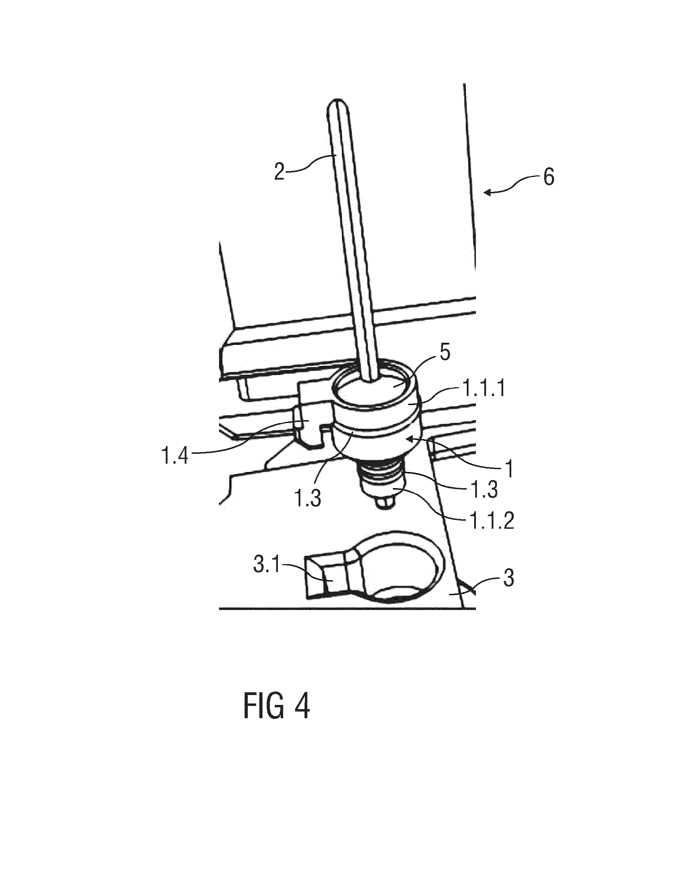

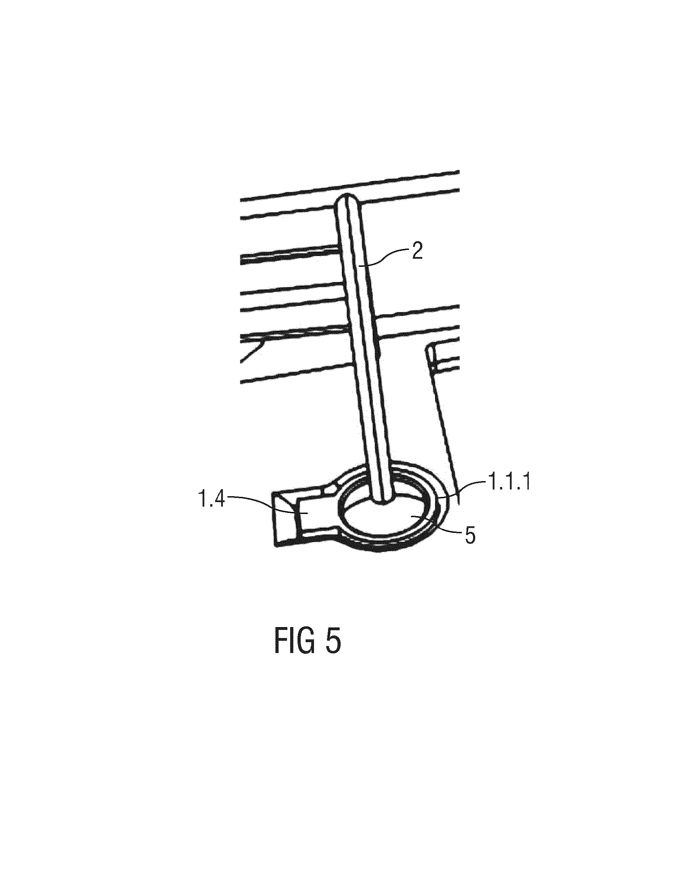

The figures 3 to 5 show an exemplary embodiment of the intermediate component 1 including the needle 2 coupled to a needle hub 3 in different views. Figure 3 shows a perspective exploded view of the needle 2, the intermediate component 1 and the needle hub 3, wherein the needle hub 3 is an integral part of a medicament delivery device 6 that is shown only in part. Figure 4 shows a further perspective exploded view, wherein the needle 2 is mounted within the intermediate component 1. Figure 5 shows the needle assembly 4, comprising the needle hub 3, the intermediate component 1 and the needle 2 in an assembled state.

The needle hub 3 comprises a latching notch 3.1 formed corresponding with the snap element 1.4 of the intermediate component 1 to generate a latching connection between the intermediate component 1 and the needle hub 3.

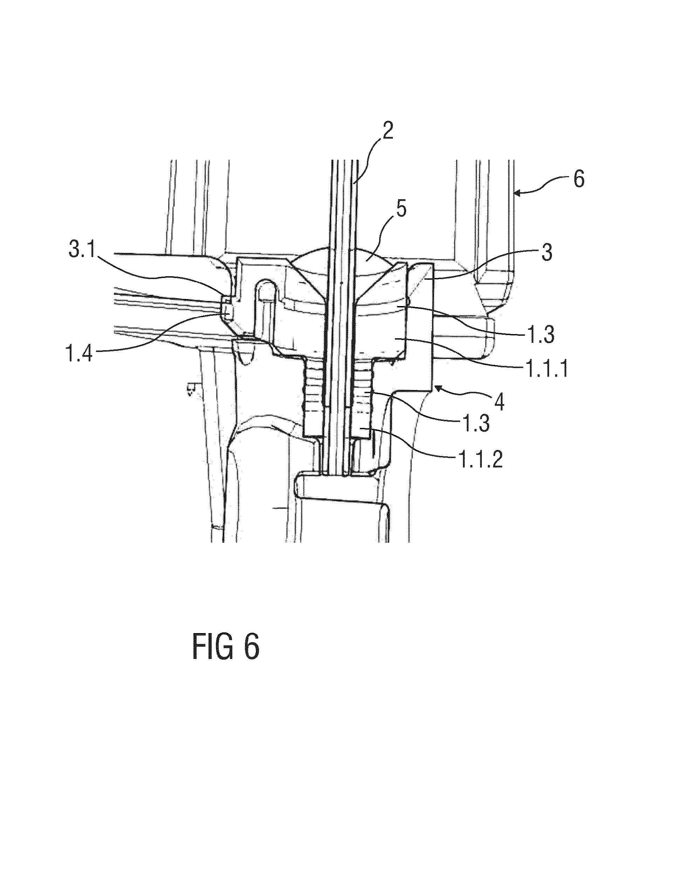

The assembled needle assembly 4 can be seen in more detail in figure 6. Figure 6 shows a longitudinal section of the assembled needle assembly 4 coupled to a component of a medicament delivery device 6, e.g. a cartridge holder.

The needle 2 is firmly bonded to the intermediate component 1 by the cured adhesive 5. The intermediate component 1 is interlockingly arranged within the needle hub 3 that is an integral part of the partly shown medicament delivery device 6. Alternatively, the needle hub 3 is a separate component that can be coupled to the medicament delivery device 6, e.g. by a snap on connection.

The intermediate component 1 is interlockingly arranged within the needle hub 3 by a latching connection between the snap element 1.4 and the latching notch 3.1. The needle assembly 4 further provides a press fitting between the intermediate component 1 and the needle hub 3 because of the arrangement of the ribs 1.3 that seals the intermediate component 1 cylindrically against the needle hub 3. Thereby, the needle 2 is fixed to the needle hub 3 without direct adhesive bonding and without contacting the hard plastic material of the needle hub 3 during the mounting of the needle assembly 4. Thus, a clogging of the needle 2 or delivery of a medicament contaminated with particles of the needle hub 3 generated by a mounting process of the needle assembly 4 is prevented.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;

or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa http://en.wikipedia.org/wiki/Dalton_%28unit%29) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by A and κ. A light chain has two successive domains: one constant domain (CL) and one variable domain (VL). The approximate length of a light chain is 211 to 217 amino acids. Each antibody contains two light chains that are always identical; only one type of light chain, κ or A, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: intermediate component
- 1.1: body
- 1.1.1: first portion
- 1.1.2: second portion
- 1.2: opening
- 1.3: rib
- 1.4: snap element
- 2: needle
- 3: needle hub
- 4: needle assembly
- 5: adhesive
- 6: medicament delivery device

a) first step
b) second step
c) third step

## Claims

1. Needle assembly (4), comprising
a needle hub (3) and
a needle (2) fixed to the needle hub (3),
**characterized in that** the needle (2) is fixed to the needle hub (3) by an intermediate component (1),
wherein the needle (2) is firmly bonded to the intermediate component (1), and wherein the intermediate component (1) is coupled to the needle hub (3) by a latching connection.

2. Needle assembly (4) according to claim 1,
**characterized in that** the intermediate component (1) is made from or comprises a soft resilient material and the needle hub (3) is made from or comprises a hard plastic material.

3. Needle assembly (4) according to claim 2,
**characterized in that** the intermediate component () is made from or comprises polyolefin.

4. Needle assembly (4) according to claim 2 or 3,
**characterized in that** the needle hub (3) is made from or comprises cyclic-olefin-copolymer.

5. Needle assembly (4) according to any one of the preceding claims,
**characterized in that** the intermediate component (1) comprises a snap element (1.4) adapted to couple the intermediate component (1) to the needle hub (3).

6. Needle assembly (4) according to any one of the preceding claims,
**characterized in that** the intermediate component (1) comprises a section which forms a press fit between the needle (2) and the intermediate component (1).

7. Needle assembly (4) according to any one of the preceding claims,
**characterized in that** the intermediate component (1) comprises a number of ribs (1.3) that are arranged on an outer circumference of the intermediate component (1) to provide a press fitting between the intermediate component (1) and the needle hub (3).

8. Needle assembly (4) according to claim 7,
**characterized in that** the ribs (1.3) are arranged ring-shaped around the outer circumference of the intermediate component (1).

9. Needle assembly (4) according to any one of the preceding claims,
**characterized in that** the needle (2) is firmly bonded to the intermediate component (1) by an adhesive (5).

10. Needle assembly (4) according to claim 9,
**characterized in that** the adhesive (5) is a light-curable adhesive.

11. Needle assembly (4) according to any one of the preceding claims,
**characterized in that** the intermediate component (1) comprises a material that transmits ultraviolet rays.

12. Method for mounting a needle assembly (4),
**characterized by** the following steps:
- prefixing a needle (2) within an intermediate component (1),
- firmly bonding the prefixed needle (2) to the intermediate component (1) by applying an adhesive (5), wherein the adhesive (5) is subsequently cured,
- snap fitting the intermediate component (1) including the needle (2) to a needle hub (3).

13. Method according to claim 12,
**characterized in that** the adhesive (5) is cured by ultraviolet rays.
